# DEMANDE DE BREVET EUROPEEN

(11) **EP 1 454 896 A1**
(43) Date de publication de la demande: **08.09.2004**
(21) Numéro de dépôt: 04290372.4
(22) Date de dépôt: 12.02.2004
(51) Int. Cl.: C07C 229/44, A61K 7/40

(54) **Nouveaux dérivés, amines, amides, sulfonamides et carbamates des sels de l'acide benzamalonique et compositions cosmétiques photoprotectrices contenant ces dérivés**

(30) Priorité: 03.03.2003 FR 0302561
(71) Demandeur: L'OREAL, 75008 Paris (FR)
(72) Inventeur: Richard, Hervé, 93420 Villerpinte (FR); Luppi, Bernadette, 77290 Mitry Mory (FR)
(74) Mandataire: Miszputen, Laurent

(57) **Abrégé**

L'invention concerne des compositions photoprotectrices, comprenant dérivés amines, amides, sulfonamides et carbamates des sels de l'acide benzalmalonique à titre de filtres solaires actifs dans le domaine des rayonnements UV.

L'invention concerne également de nouveaux dérivés amines, amides, sulfonamides et carbamates des sels de l'acide benzalmalonique et leurs utilisations.

## Description

L'invention concerne des compositions photoprotectrices, comprenant dérivés amines, amides, sulfonamides et carbamates des sels de l'acide benzalmalonique à titre de filtres solaires actifs dans le domaine des rayonnements UV.

L'invention concerne également de nouveaux dérivés amines, amides, sulfonamides et carbamates des sels de l'acide benzalmalonique et leurs utilisations.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel. Pour ces raisons ainsi que pour des raisons esthétiques, il existe une demande croissante de moyens de contrôle de ce bronzage naturel. Il convient donc de filtrer ce rayonnement UV-B.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

Les filtres organiques sont le plus souvent formulés dans des compositions présentent sous forme d'émulsions huile-dans-eau ou eau-dans-huile Les filtres organiques, généralement lipophiles ou hydrophiles, sont présents sous forme dissoute, dans l'une ou l'autre de ces phases, en des quantités appropriées pour obtenir le facteur de protection solaire (FPS) recherché.

On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

Outre leur pouvoir filtrant du rayonnement solaire, les composés photoprotecteurs doivent également présenter de bonnes propriétés cosmétiques, une bonne solubilité dans les solvants usuels et notamment dans les milieux aqueux et une photostabilité satisfaisante.

La demanderesse a découvert de manière surprenante une nouvelle famille de dérivés amines, amides, sulfonamides et carbamates des sels de l'acide benzalmalonique présentant des propriétés d'absorption dans la zone des radiations UV-B. Ces composés peuvent être incorporés dans des formulations cosmétiques. Ils présentent une bonne solubilité dans les milieux aqueux, une bonne photostabilité et présentent des qualités cosmétiques satisfaisantes.

L'un des objets de la présente invention, consiste en de nouveaux dérivés amines, amides, sulfonamides et carbamates des sels de l'acide benzalmalonique répondant à la formule suivante (1) ou (2) que l'on définira plus en détail par la suite et leurs utilisations.

L'invention concerne également une composition cosmétique ou dermatologique, destinée à la photoprotection des matières kératiniques, contenant dans un milieu cosmétiquement acceptable au moins un composé de formule (1) ou (2).

D'autres objets apparaîtront à la lumière de la description.
Les composés conformes à la présente invention répondent à la formule générale (1) ou (2) suivante : 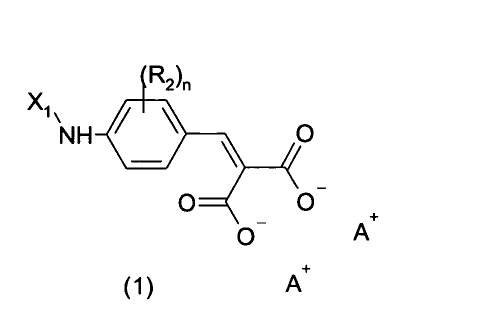 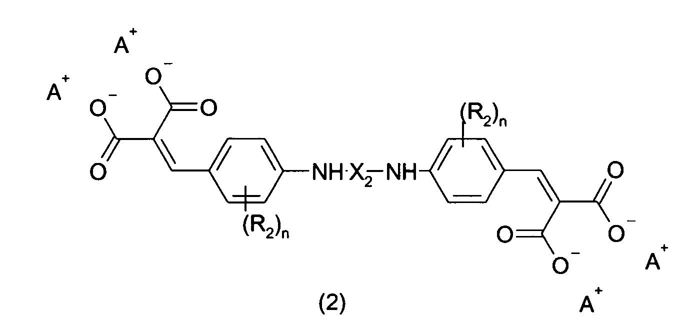 dans lesquelles
X₁ représente l'hydrogène ou un radical R₃-(C=O)- , R₃-SO₂- ou R₃-O-(C=O)-,
X₂ représente un radical divalent de formule -(C=O)-R'₃-(C=O)- ,
-SO₂-R'₃-SO₂- ou -(C=O)-O-R'₃-O-(C=O)-,
R₂ représente un groupe alkyle en C₁₋₈, linéaire ou ramifié,
n vaut 0, 1 ou 2,
R₃ représente un radical alkyle en C₁-C₃₀ ou alcényle en C₃-C₃₀, linéaire ou ramifié, pouvant porter un ou plusieurs substituants hydroxyle et pouvant contenir dans la chaîne carbonée un ou plusieurs hétéroatomes choisis parmi les atomes d'oxygène, d'azote et de silicium, un radical aryle en C₆-C₂₀, éventuellement substitué ;
R'₃ représente une simple liaison ou un radical divalent alkylène en C₁-C₃₀ ou alcénylène en C₃-C₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,
les radicaux A, identiques ou différents représentent un cation du type métal alcalin comme sodium ou potassium ; un groupe ammonium ; un radical mono, di- ou tri- alkylammonium en C₁-C₂₀ ; un radical mono, di- ou trialkanolammonium en C₂-C₂₀ ; un groupe héterocyclique azoté quaternaire en C₅-C₈.

Dans la formule (I) ci-dessus, les radicaux alkyles peuvent être choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et tert.-octyle. Le radical alkyle particulièrement préféré est le radical méthyle.

Dans les formules (I) ci-dessus, les radicaux alcoxy peuvent être choisis notamment au sein des radicaux méthoxy, éthoxy, n-propoxy, isopropoxy, n-butoxy et isobutoxy. Le radical alcoxy particulièrement préféré est le radical méthoxy.

Dans la formule (I) ci-dessus, les radicaux alcènyle peuvent être choisis notamment au sein des radicaux propylène, butène.

Dans la formule (I) ci-dessus, les radicaux aryle sont par exemple benzyle ou phényle

Dans la formule (I) ci-dessus, les radicaux mono, di ou trialkylammonium en C₁-C₂₀ peuvent être choisis parmi mono, di- ou triméthylammomnium ; ou mono, di- ou triéthylammomnium

Dans la formule (I) ci-dessus, les radicaux mono, di ou trialcanolammonium en C₂-C₂₀ peuvent être choisis parmi mono, di- ou triéthanolammonium,

Dans la formule (I) ci-dessus, les radicaux hétérocycles quaternaires peuvent être choisi parmi pipéridinium, morpholinium, pyrrolidinium, ou pyrrolinium.

Parmi les composés de formule (I), on citera encore plus particulièrement ceux de formule (1) pour lesquels :
X₁ est hydrogène ; R₃-(C=O)-
n = 0
R₃ désigne alkyle ;
A désigne un métal alcalin ou un groupe pipéridinium.

Parmi les composés de formule (I), on citera encore plus particulièrement ceux choisis parmi :

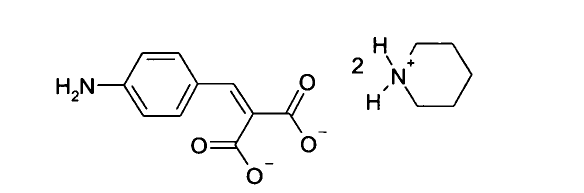

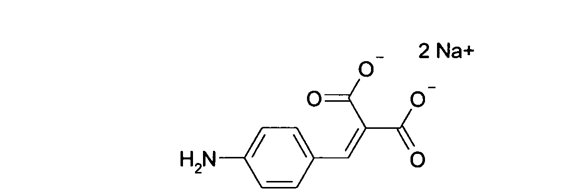

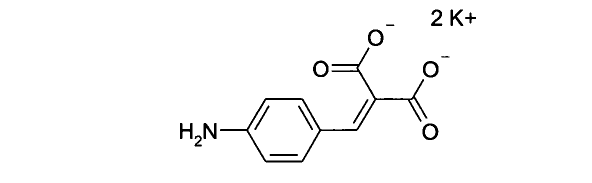

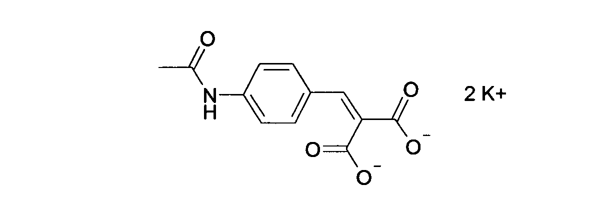

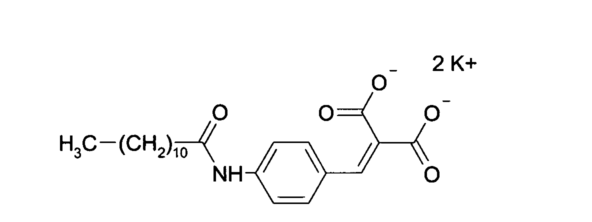

Les dérivés amides, sulfonamides et carbamates des sels de l'acide benzalmalonique de formule (1) peuvent être obtenus par les deux voies de synthèse suivantes :
Voie A : obtention des dérivés amides, sulfonamides et carbamates par condensation du para amino benzaldéhyde (3) sur les chlorures d'acide, sulfochlorures et chloroformiates (4) correspondants selon le schéma (I) suivant : 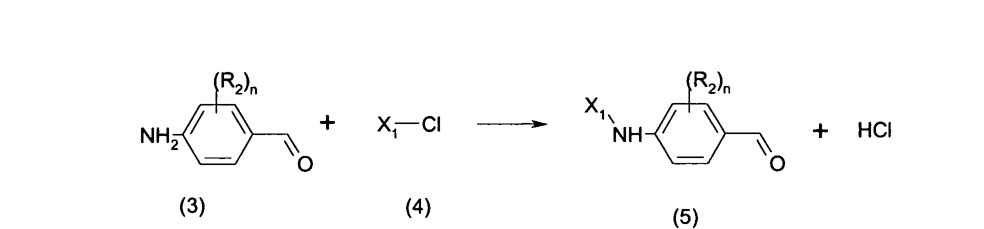 suivi de la condensation du benzaldéhyde (5) sur le di-sel de l'acide malonique selon le schéma (II) suivant : 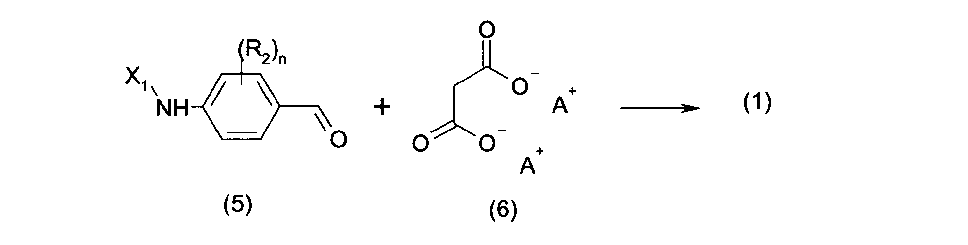
Voie B : obtention du di-sel de l'acide para amino benzalmalonique (6) par condensation du para amino benzaldéhyde (3) sur le di-sel de l'acide malonique (6) selon le schéma (III) suivant : 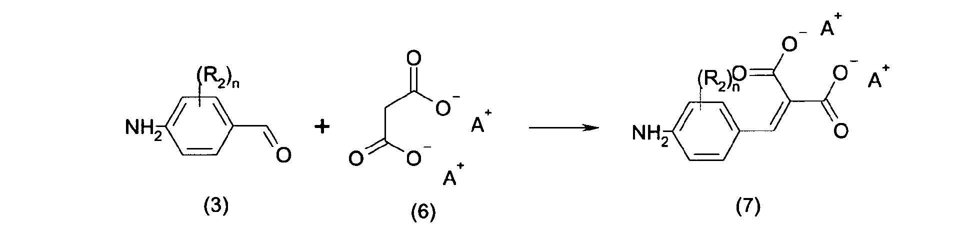 suivi de la réaction des sels de l'acide para amino benzalmalonique sur les chlorures d'acide, sulfochlorures et chloroformiates correspondants selon le schéma (IV) suivant :

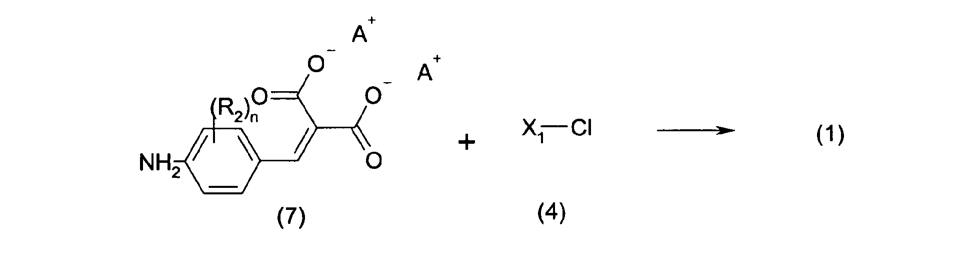

Un autre objet de la présente invention est la synthèse nouvelle directe des sels de l'acide para amino malonique par le procédé schématisé par le schéma (III) ci-dessus.

Cette synthèse s'effectue de manière générale sans solvant ou en présence d'un solvant alcoolique tel le méthanol, l'éthanol ou l'alcool isopropylique et à une température comprise entre 40 et 120°C et plus particulièrement entre 60 et 95°C.

Les composés de formule (I) sont généralement présents dans la composition de l'invention dans des proportions comprises entre 0,01 % et 20 % en poids, de préférence entre 0,1 % et 10 % en poids, par rapport au poids total de la composition.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4,367,390, EP863145, EP517104, EP570838, EP796851, EP775698, EP878469, EP933376, EP507691, EP507692, EP790243, EP944624 ; les dérivés de la benzophénone; les dérivés de β,β-diphénytacrytate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE19726184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCI :
Dérivés de l'acide para-aminobenzoique :
   PABA,
   Ethyl PABA,
   Ethyl Dihydroxypropyl PABA,
   Ethylhexyl Diméthyl PABA vendu notamment sous le nom « ESCALOL 507 » par ISP,
   Glyceryl PABA,
   PEG-25 PABA vendu sous le nom « UVINUL P25 » par BASF,
Dérivés salicyliques:
   Homosalate vendu sous le nom « Eusolex HMS » par Rona/EM Industries,
   Ethylhexyl Salicylate vendu sous le nom « NEO HELIOPAN OS » par HAARMANN et REIMER,
   Dipropyleneglycol Salicylate vendu sous le nom « DIPSAL » par SCHER,
   TEA Salicylate, vendu sous le nom « NEO HELIOPAN TS » par HAARMANN et REIMER,
Dérivés du dibenzoylméthane :
   Butyl Methoxydibenzoylmethane vendu notamment sous le nom commercial « PARSOL 1789 » par HOFFMANN LA ROCHE,
   Isopropyl Dibenzoylmethane,
Dérivés cinnamiques :
   Ethylhexyl Methoxycinnamate vendu notamment sous le nom commercial « PARSOL MCX » par HOFFMANN LA ROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   - Diisopropyl Methylcinnamate,
   Glyceryl Ethylhexanoate Dimethoxycinnamate
Dérivés de β,β'-diphénylacrylate :
   Octocrylene vendu notamment sous le nom commercial « UVINUL N539 » par BASF,
   Etocrylene, vendu notamment sous le nom commercial « UVINUL N35 » par
   BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF, Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF, Benzophenone-12
   2-(4-diethylamino-2-hydroxybenzoyl)-benzoate de n-hexyle.
Dérivés du benzylidène camphre :
   3-Benzylidene camphor fabriqué sous le nom « MEXORYL SD» par CHIMEX,
   4-Methylbenzylidene camphor vendu sous le nom « EUSOLEX 6300 » par MERCK,
   Benzylidene Camphor Sulfonic Acid fabriqué sous le nom « MEXORYL SL» par CHIMEX,
   Camphor Benzalkonium Methosulfate fabriqué sous le nom « MEXORYL SO » par CHIMEX,
   Terephthalylidene Dicamphor Sulfonic Acid fabriqué sous le nom « MEXORYL SX » par CHIMEX,
   Polyacrylamidomethyl Benzylidene Camphor fabriqué sous le nom « MEXORYL SW » par CHIMEX,
Dérivés du phenyl benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par HAARMANN et REIMER,
Dérivés de la triazine :
   Anisotriazine vendu sous le nom commercial «TINOSORB S » par CIBA GEIGY,
   Ethylhexyl triazone vendu notamment sous le nom commercial «UVINUL T150 » par BASF,
   2,4,6-tris-(4'-amino benzalmalonate de diisobutyle)-s- triazine,
   Diethylhexyl Butamido Triazone vendu sous le nom commercial « UVASORB HEB » par SIGMA 3V,
Dérivés du phenyl benzotriazole :
   Drometrizole Trisiloxane vendu sous le nom « Silatrizole » par RHODIA CHIMIE
   Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par HAARMANN et REIMER,
Dérivés d'imidazolines :
   Ethylhexyl Dimethoxybenzylidene Dioxoimidazoline Propionate,
Dérivés du benzalmalonate :
   Polyorganosiloxane à fonctions benzalmalonate vendu sous la dénomination commerciale « PARSOL SLX » par HOFFMANN LA ROCHE
Dérivés de 4,4-diarylbutadiène :
   - 1,1-dicarboxy (2,2'-diméthyl-propyl)-4,4-diphénylbutadiène
   et leurs mélanges.

Les filtres UV organiques complémentaires préférentiels sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
-1,1-dicarboxy (2,2'-diméthyl-propyl)-4,4-diphénylbutadiène et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres UV complémentaires conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions cosmétiques selon l'invention peuvent contenir en outre des agents de bronzage et/ou de brunissage artificiel de la peau (agents autobronzants) tels que la dihydroxyacétone (DHA).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de camauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les polymères acryliques réticulés comme les Carbomer, les polymères réticulés acrytates/C₁₀-C₃₀ alkylacrylates du type Pemulen ou le polyacrylate-3 vendu sous le nom VISCOPHOBE DB 1000 par Amerchol ; les polyacrylamides tels que l'émulsion polyacrylamide, C₁₃-C₁₄ isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Bien entendu, l'homme de l'art veillera à choisir le ou les éventuels composés complémentaires cités ci-dessus et/ou leurs quantités de manière telle que les propriétés avantageuses attachées intrinsèquement aux composés conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, d'une huile, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, d'huile solaire, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Un autre objet de l'invention est l'utilisation d'un composé de formule (1) ou (2) tel que défini ci-dessus dans une composition cosmétique ou dermatologique comme agent filtrant les radiations UV.

Un autre objet de l'invention est l'utilisation d'un composé de formule (1) ou (2) tel que défini ci-dessus dans une composition cosmétique comme agent de contrôle de la variation de la couleur de la peau due aux rayonnements UV.

Un autre objet de l'invention est l'utilisation d'un composé de formule (1) ou (2) tel que défini précédemment comme agent photostabilisant de polymères synthétiques tels que les matières plastiques ou de verres en particulier des verres de lunettes ou des lentilles de contact.

Des exemples concrets, mais nullement limitatifs, illustrant l'invention, vont maintenant être donnés.

### EXEMPLE 1 : Préparation du sel de di-pépiridinium de l'acide 2-(4-amino-benzylidène)-malonique:

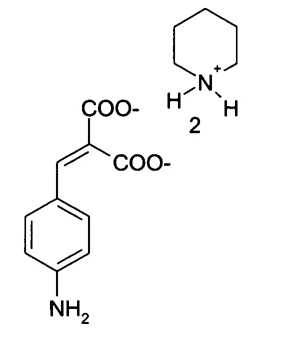

Dans un réacteur équipé d'un thermomètre, d'une agitation mécanique et d'un réfrigérant, on place la pipéridine (206,6g, 2,42 mole). On chauffe vers 40°C. On introduit l'acide malonique par portions en 15 minutes sans dépasser 70°C (83,4g, 0,8 mole). On maintient la pâte visqueuse obtenue à 70°C et introduit par portions le p-amino benzaldéhyde en 15 minutes (97g, 0,8 mole). Le mélange visqueux est laissé à 90°C sous agitation et contrôle de viscosité. Lorsque la viscosité devient plus importante, on commence à introduire l'alcool isopropylique (après 1 heure 15 minutes). L'introduction de 200 ml d'alcool isopropylique s'est faite en 2 heures 50 minutes. On refroidit et on filtre. Le précipité obtenu est lavé avec un minimum d'alcool isopropylique. Après séchage sous vide sur P₂O₅, on obtient 210 g (Rendement 70 %) du produit de l'exemple 1 sous forme de poudre jaune :

| | | | |
|---|---|---|---|
| UV (Eau) | λₘₐₓ = 305 nm | εₘₐₓ = 16 840 | E_{1%} = 446 |

### EXEMPLE 2 : Préparation du sel di-sodique de l'acide 2-(4-aminobenzylidène)-malonique:

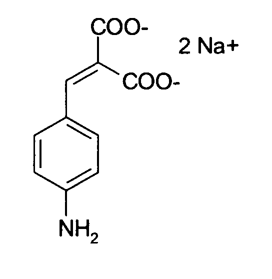

Le mode opératoire de l'exemple 1 est reproduit jusqu'à l'étape d'addition d'alcool isopropylique. Ensuite, on introduit de la soude (200ml de soude aqueuse à 35%, 1,75 mole) en 15 minutes à une température de 90°C-70°C toujours sous agitation. On refroidit vers 40°C. On ajoute 700 ml d'éthanol et filtre le mélange réactionnel sur Fritté N°3. Le solide est repris dans au moins 500 ml d'eau et filtré. On obtient environ 5 g de résidu que l'on jette. Le filtrat est concentré de moitié et on ajoute 700 ml d'éthanol à 95 et laisse cristalliser. Après filtration, séchage sous vide sur P₂O₅, on obtient 140 g (Rendement 70 %) du produit de l'exemple 2 sous forme de poudre jaune :

| | | | |
|---|---|---|---|
| UV (Eau) | λₘₐₓ = 305 nm | εₘₐₓ = 16 830 | E_{1%} = 670 |

### EXEMPLE 3 : Préparation du sel di-potassique de l'acide 2-(4-aminobenzylidène)-malonique:

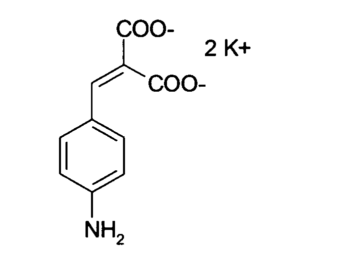

Le mode opératoire de l'exemple 1 est reproduit jusqu'à l'étape d'addition d'alcool isopropylique. Ensuite, on introduit de la potasse à 85% (114g, 1,75 mole) dissoute dans 200ml d'éthanol à 96) en 15 minutes à une température de 90°C-70°C toujours sous agitation. On refroidit vers 40°C. On ajoute 700 ml d'éthanol à 96 et filtre le mélange réactionnel sur Fritté N°3. Le solide est repris dans au moins 500 ml d'eau et filtré. Le filtrat est concentré de moitié et on ajoute 500 ml d'isopropanol et laisse cristalliser. Après filtration, séchage sous vide sur P₂O₅, on obtient 205g (Rendement 90 %) du produit de l'exemple 3 sous forme de poudre jaune clair :

| | | | |
|---|---|---|---|
| UV (Eau) | λₘₐₓ = 306 nm | εₘₐₓ=14050 | E_{1%}=496 |

### EXEMPLE 4 : Préparation du sel di-potassique de l'acide 2-(4-acétamido-benzylidène)-malonique:

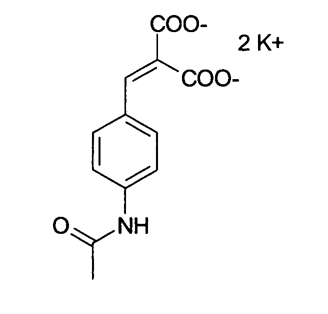

Dans un réacteur équipé d'un thermomètre, d'une agitation mécanique et d'un réfrigérant, on place la pipéridine (30ml). On chauffe vers 40°C. On introduit l'acide malonique par portions en 15 minutes sans dépasser 70°C (10,4g, 0,1 mole). On introduit par portions le p-acétamido benzaldéhyde (16,3g, 0,1 mole). Le mélange est laissé à 95°C sous agitation et contrôle de viscosité.
Lorsque la viscosité devient plus importante, on commence à introduire l'alcool isopropylique (après 1 heure). L'introduction de 20 ml d'alcool isopropylique s'est faite en 1 heure. On refroidit. On ajoute la potasse (15g) dissoute dans 80ml d'éthanol à 96. Le précipité obtenu est filtré, lavé avec un minimum d'éthanol à 96 puis séché sous vide. On obtient 27,9 g (Rendement 86 %) du produit de l'exemple 4 sous forme de poudre blanche :

| | | | |
|---|---|---|---|
| UV (Eau) | λₘₐₓ = 292 nm | εₘₐₓ = 16 220 | E_{1%} = 498 |

### EXEMPLE 5 : Préparation du sel di-potassique de l'acide 2-(4-laurylamido-benzylidène)-malonique:

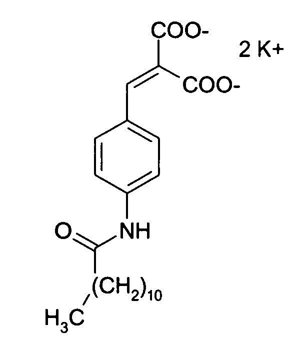

### Première étape : Préparation du para laurylamido benzaldéhyde :

Dans un réacteur équipé d'un thermomètre, d'une agitation mécanique et d'un réfrigérant, on place le para amino benzaldéhyde (12,1g, 0,1 mole) en suspension dans 100ml de dichloro-1,2 éthane. On introduit le chlorure d'acide laurique (21,8g, 0,1 mole) puis la triéthylamine (10,1g, 0,1 mole). Le mélange est chauffé au reflux du dichloro-1,2 éthane pendant 4 heures. On refroidit, filtre le para amino benzaldéhyde n'ayant pas réagi. Après concentration du filtrat, le résidu est chromatographié sur silice (éluant CH₂Cl₂ puis CH₂Cl₂-MeOH 95 :5) on obtient 3 g (Rendement 10 %) de fractions propres de para laurylamido benzaldéhyde sous forme d'une poudre blanche utilisée telle quelle dans l'étape suivante :

### Deuxième étape : Préparation du dérivé de l'exemple 5 :

Dans un réacteur équipé d'un thermomètre, d'une agitation mécanique et d'un réfrigérant, on place la pipéridine (1g). On chauffe vers 40°C. On introduit l'acide malonique (0,5g). On introduit le para laurylamido benzaldéhyde (1g, 3x10-3 mole) et 2 ml d'alcool isopropylique. Le mélange est laissé 2 heures à 90°C sous agitation. On refroidit. On ajoute la potasse (1g) dissoute dans 10ml d'éthanol à 96. Le précipité obtenu est filtré, lavé avec un minimum d'éthanol à 96 puis séché sous vide. On obtient 0,6 g (Rendement 45 %) du produit de l'exemple 5 sous forme d'un solide cireux blanc :

| | | | |
|---|---|---|---|
| UV (Eau) | λₘₐₓ = 292 nm | εₘₐₓ = 14 010 | E_{1%} = 422 |

### EXEMPLE 6: Composition antisolaire (émulsion huile-dans-eau)

- composé de l'exemple 4 3 g
- Mélange d'alcool cétylstéarylique et d'alcool cétylstéarylique oxyéthyléné (33 motifs OE) 80/20 vendu par la société TENSIA sous la dénomination commerciale DEHSCONET® 390 7 g
- Mélange de mono- et de distéarate de glycérol vendu sous la dénomination commerciale CERASYNTH® SD par la société ISP 2 g
- Alcool cétylique 1,5 g
- Polydiméthylsiloxane vendu sous la dénomination DC200Fluid® par la société DOW CORNING 1,5 g
- Glycérine 15 g
- Conservateurs q.s.
- Eau déminéralisée qsp 100 g

La phase grasse est chauffée à environ 70 - 80 °C jusqu'à fusion complète. On ajoute ensuite sous agitation vigoureuse la phase aqueuse contenant le composé de l'exemple 4 en une seule fois à 80°C. On maintient l'agitation pendant 10 à 15 minutes, puis on laisse refroidir sous agitation modérée jusqu'à environ 40 °C et on ajoute les conservateurs. On obtient ainsi une crème antisolaire particulièrement efficace contre les UV-B.

## Revendications

1. Composé répondant à la formule générale (1) ou (2) suivante : 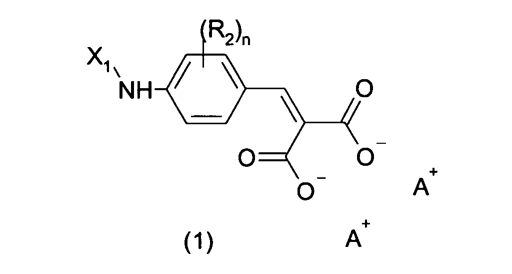 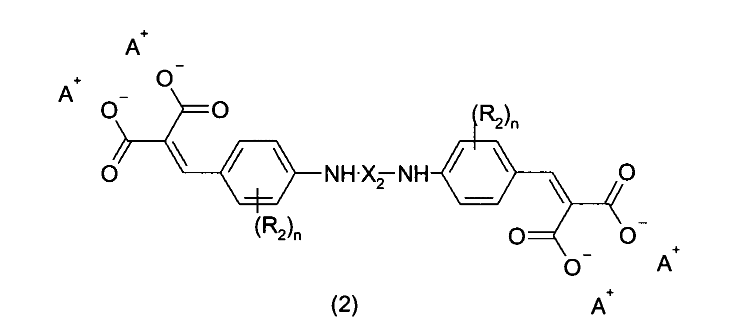 dans lesquelles
X₁ représente l'hydrogène ou un radical R₃-(C=O)- , R₃-SO₂- ou R₃-O-(C=O)-,
X₂ représente un radical divalent de formule -(C=O)-R'₃-(C=O)- ,
-SO₂-R'₃-SO₂- ou -(C=O)-O-R'₃-O-(C=O)-,
R₂ représente un groupe alkyle en C₁₋₈, linéaire ou ramifié,
n vaut 0, 1 ou 2,
R₃ représente un radical alkyle en C₁-C₃₀ ou alcényle en C₃-C₃₀, linéaire ou ramifié, pouvant porter un ou plusieurs substituants hydroxyle et pouvant contenir dans la chaîne carbonée un ou plusieurs hétéroatomes choisis parmi les atomes d'oxygène, d'azote et de silicium, un radical aryle en C₆-C₂₀, éventuellement substitué ;
R'₃ représente une simple liaison ou un radical divalent alkylène en C₁-C₃₀ ou alcénylène en C₃-C₃₀, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,
les radicaux A, identiques ou différents représentent un cation du type métal alcalin comme sodium ou potassium ; un groupe ammonium ; un radical mono, di- ou tri- alkylammonium en C₁-C₂₀ ; un radical mono, di- ou trialkanolammonium en C₂-C₂₀ ; un groupe héterocyclique azoté quaternaire en C₅-C₈ ; à l'exception de l'acide 2-(4-acétamido-benzylidène)-malonique:

2. Composé selon la revendication 1, de formule (1), pour lequel :
X₁ est hydrogène ; R₃-(C=O)-
n = 0
R₃ désigne alkyle ;
A désigne un métal alcalin ou un groupe pipéridinium.

3. Composé selon la revendication 2, choisi parmi les composés suivants :

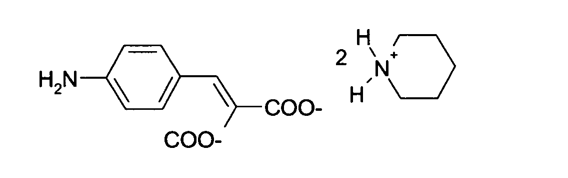

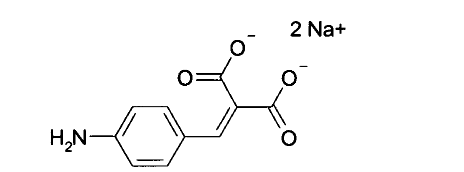

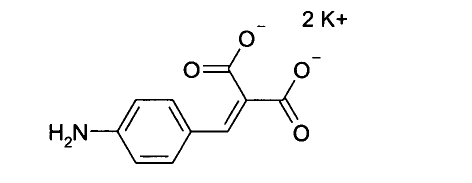

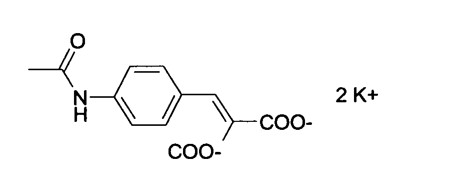

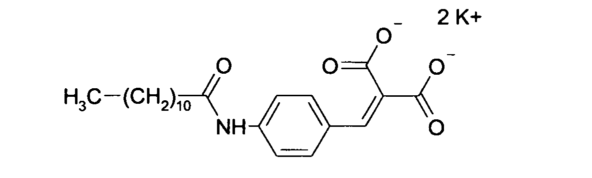

4. Procédé de préparation d'un composé de formule (1) tel que défini dans l'une quelconque des revendications précédentes, qui consiste a obtenir dans une première étape un di-sel de l'acide para amino benzalmalonique (7) par condensation du para amino benzaldéhyde (3) sur le di-sel de l'acide malonique (6) selon le schéma suivant : 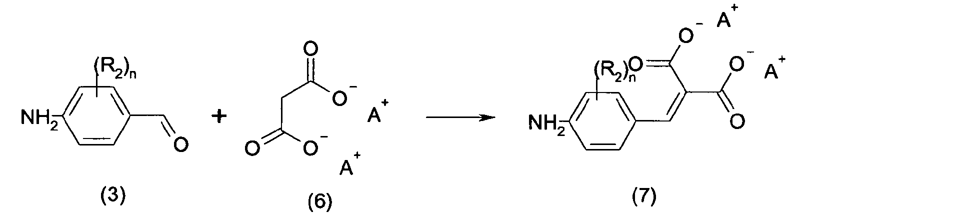 puis dans une deuxième étape à faire réagir le di-sel de l'acide para amino benzalmalonique (7) ainsi obtenu sur un chlorure d'acide, sulfochlorure ou chloroformiate de formule (4)selon le schéma suivant : 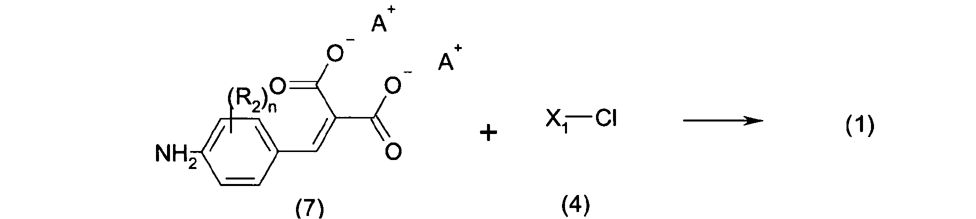 les radicaux R₂, n, A, X₁ ayant les mêmes indications indiquées dans les formules (1) ou (2) dans les revendications précédentes.

5. Procédé selon la revendication 4, où les conditions de mise en oeuvre de la première étape sont les suivantes : la synthèse s'effectue de manière générale sans solvant ou en présence d'un solvant alcoolique tel le méthanol, l'éthanol ou l'alcool isopropylique et à une température comprise entre 40 et 120°C et plus particulièrement entre 60 et 95°C.

6. Composition cosmétique ou dermatologique, destinée à la photoprotection des matières kératiniques, **caractérisée par le fait qu'**elle contient dans un support cosmétiquement acceptable au moins un composé répondant à la formule (1) ou (2) tel que défini dans l'une quelconque des revendications 1 à 3.

7. Composition selon la revendication 6, **caractérisée par le fait que** le composé de formule (1) ou (2) est présent dans la composition à une teneur allant de 0,01 à 20 % en poids, par rapport au poids total de la composition, de préférence allant de 0,1 % à 10 % en poids, par rapport au poids total de la composition.

8. Composition selon la revendication 6 ou 7, **caractérisée par le fait que** ledit support cosmétiquement acceptable se présente sous la forme d'une émulsion de type huile-dans-eau ou eau-dans-huile.

9. Composition selon l'une quelconque des revendications 6 à 8, **caractérisée par le fait qu'**elle comprend en outre un ou plusieurs filtres organiques ou inorganiques complémentaires actifs dans l'UV-A et/ou UV-B.

10. Composition selon la revendication 9, **caractérisée par le fait que** lesdits filtres organiques complémentaires sont choisis parmi les anthranilates ; les dérivés cinnamiques ; les dérivés de dibenzoylméthane ; les dérivés salicyliques, les dérivés du camphre ; les dérivés de triazine; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres; les dimères dérivés d'α-alkylstyrène; les 4,4-diarylbutadiènes et leurs mélanges.

11. Composition selon la revendication 10, **caractérisée par le fait que** lesdits filtres organiques complémentaires sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
1,1-dicarboxy (2,2'-diméthyl-propyl)-4,4-diphénylbutadiène
et leurs mélanges.

12. Composition selon la revendication 9, **caractérisée par le fait que** les filtres complémentaires inorganiques sont des pigments ou des nanopigments d'oxydes métalliques, enrobés ou non.

13. Composition selon la revendication 12, **caractérisée par le fait que** lesdits pigments ou nanopigments sont choisis parmi les oxydes de titane, de zinc, de fer, de zirconium, de cérium et leurs mélanges, enrobés ou non.

14. Composition selon l'une quelconque des revendications 1 à 13, **caractérisée par le fait qu'**elle comprend en outre au moins un agent de bronzage et/ou de brunissage artificiel de la peau.

15. Composition selon l'une quelconque des revendications 1 à 14, **caractérisée par le fait qu'**elle comprend en outre au moins un adjuvant choisi parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants

16. Composition selon l'une quelconque des revendications 1 à 15, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une huile, d'une poudre, d'un bâtonnet solide, d'une mousse ou d'un spray.

17. Composition selon l'une quelconque des revendications 1 à 15, **caractérisée par le fait qu'**il s'agit d'une composition de maquillage des cils, des sourcils, des ongles ou de la peau et qu'elle se présente sous forme solide ou pâteuse, anhydre ou aqueuse, d'une émulsion, d'une suspension ou d'une dispersion.

18. Composition selon l'une quelconque des revendications 1 à 16, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

19. Utilisation d'un composé de formule (1) ou (2) tel que défini dans l'une quelconque des revendications 1 à 3 dans une composition cosmétique ou dermatologique comme agent filtrant les radiations UV.

20. Utilisation d'un composé de formule (1) ou (2) tel que défini dans l'une quelconque des revendications 1 à 3 dans une composition cosmétique comme agent de contrôle de la variation de la couleur de la peau due aux rayonnements UV.

21. Utilisation d'un composé de formule (1) ou (2) tel que défini dans l'une quelconque des revendications 1 à 3 comme agent photostabilisant de polymères synthétiques ou de verres.
